# EUROPEAN PATENT APPLICATION

(11) **EP 4 509 149 A1**
(43) Date of publication of application: **19.02.2025**
(21) Application number: 23788641.1
(22) Date of filing: 14.04.2023
(51) Int. Cl.: A61M 5/142, A61M 5/168, A61M 5/158, A61M 5/172, A61B 5/145

(54) **MEDICINAL LIQUID INJECTION DEVICE**

(30) Priority: 15.04.2022 US 202263363086 P
(71) Applicant: Eoflow Co., Ltd., Seongnam-si, Gyeonggi-do 13605 (KR)
(72) Inventor: WELSFORD, Ian G., Stratham, New Hampshire 03885 (US); NEFF, James, Burlington, Massachusetts 01803-4261 (US); FLINT, Neave, Berwick, Maine 03901 (US)
(74) Representative: Meissner Bolte Partnerschaft mbB
(86) International application number: PCT/KR2023/005083
(87) International publication number: WO 2023/200292

(57) **Abstract**

A medical liquid infusion apparatus includes a body portion in which a plurality of needle hole portions are formed, a needle assembly connected to the needle hole portions and being capable of contacting a user's body, a substrate capable of applying voltage to the needle assembly, and a pressure generation unit configured to provide pressure to discharge medical liquid accommodated in the needle assembly to outside.

## Description

### Technical Field

The present disclosure relates to a medical liquid infusion apparatus.

### Background Art

Generally, a medical liquid infusion apparatus such as an insulin infusion apparatus is used to infuse medical liquid into a body of a patient, and is used by medical professionals like doctors or nurses, but in most cases, by ordinary persons like patients or carers for the patients.

For diabetic patients, especially, pediatric diabetic patients, medicine like insulin needs to be infused into a human body at set intervals.

A medical liquid infusion apparatus in the form of a patch used by being attached to a human body for a specific period has been developed, and such a medical liquid infusion apparatus may be used by being attached to the human body such as an abdomen, waist, etc., of a patient, in the form of a patch for the specific period.

The medical liquid infusion apparatus, when attached to the human body, needs to be comfortable to wear, convenient to use, durable, and driven with low power.

In particular, the medical liquid infusion apparatus is used by being directly attached to the skin of the patient, such that it is important for a user to run the medical liquid infusion apparatus conveniently and safely.

To increase the effect of medicine infusion, the medical liquid infusion apparatus needs to be controlled to precisely infuse a required amount of medicine into the body of the patient, and it is important to precisely infuse a small amount of medicine through a small-size medical liquid infusion apparatus.

Medicine has been conventionally infused into the body using a driving device such as a piston, etc., but this type of medicine infusion apparatus is difficult to control for precise infusion of a required amount of medicine.

### Disclosure

### Technical Problem

The present disclosure provides a medical liquid infusion apparatus deforming a shape of a needle assembly by controlling voltage provided from a substrate, thereby precisely controlling the amount of medical liquid to be infused to a user.

### Technical Solution

According to an aspect of the present disclosure, a medical liquid infusion apparatus includes a body portion in which a plurality of needle hole portions are formed, a needle assembly connected to the needle hole portions and being capable of contacting a user's body, a substrate capable of applying voltage to the needle assembly, and a pressure generation unit configured to provide pressure to discharge medical liquid accommodated in the needle assembly to outside.

The needle assembly may include an infusion needle portion capable of infusing medicine into a user's body and a sensing needle portion separated from the infusion needle portion and capable of collecting user's body data.

The needle assembly may further include a variable member capable of contacting the substrate and being shape-deformed by receiving voltage from the substrate.

The variable member may receive voltage to communicate the infusion needle portion with the pressure generation unit.

The substrate may include a processor configured to receive the body data from the sensing needle portion and generate a control signal and a first electrode configured to receive the control signal and apply voltage to the variable member.

The processor may be further configured to receive an electric signal from an external user terminal and control the first electrode to selectively apply voltage to the variable member based on the electric signal.

### Advantageous Effects

A medical liquid infusion apparatus according to an embodiment of the present disclosure may control medicine infusion to a user by applying or removing, by a substrate, voltage to or from a needle assembly.

In addition, as the substrate may receive user's body data from the needle assembly to control the voltage applied to the needle assembly, a medical liquid infusion environment optimized for a user's body state may be provided.

### Description of Drawings

FIG. 1 is a perspective view of a medical liquid infusion apparatus according to an embodiment of the present disclosure.
FIG. 2 is an exploded view of a medical liquid infusion apparatus according to an embodiment of the present disclosure.
FIG. 3 is a cross-sectional view cut along a line A-A' of FIG. 1.
FIG. 4 is a view schematically showing a portion B of FIG. 3.
FIGS. 5A and 5B are diagrams showing use states of a medical liquid infusion apparatus according to an embodiment of the present disclosure.
FIG. 6 is a plan view of a pressure generation unit according to another embodiment of the present disclosure.
FIG. 7 is a view showing flow of fluid in a pressure generation unit according to another embodiment of the present disclosure.
FIG. 8 is a bottom perspective view showing a medical liquid infusion apparatus according to another embodiment of the present disclosure.

### BEST MODE

The present disclosure may have various modifications thereto and various embodiments, and thus particular embodiments will be illustrated in the drawings and described in detail in a detailed description. Effects and features of the present disclosure, and a method of achieving them will be apparent with reference to the embodiments described in detail in conjunction with the drawings. However, the present disclosure is not limited to the embodiments disclosed below, but may be implemented in various forms.

Hereinafter, embodiments of the present disclosure will be described in detail with reference to the accompanying drawings, and in description with reference to the drawings, the same or corresponding components are given the same reference numerals, and redundant description thereto will be omitted.

In the following embodiments, singular forms include plural forms unless apparently indicated otherwise contextually.

In the following embodiments, the terms "include", "have", or the like, are intended to mean that there are features, or components, described herein, but do not preclude the possibility of adding one or more other features or components.

When a certain embodiment may be implemented otherwise, a particular process order may be performed differently from the order described. For example, two processes described in succession may be performed substantially simultaneously, or may be performed in an order reverse to the order described.

In the drawings, the size of components may be exaggerated or reduced for convenience of description. For example, the size and thickness of each component shown in the drawings are shown for convenience of description, and thus the present disclosure is not necessarily limited to the illustration.

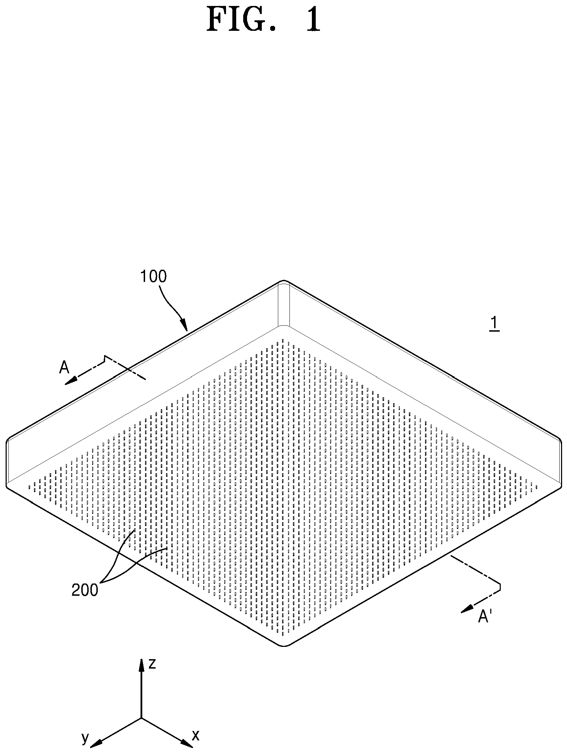

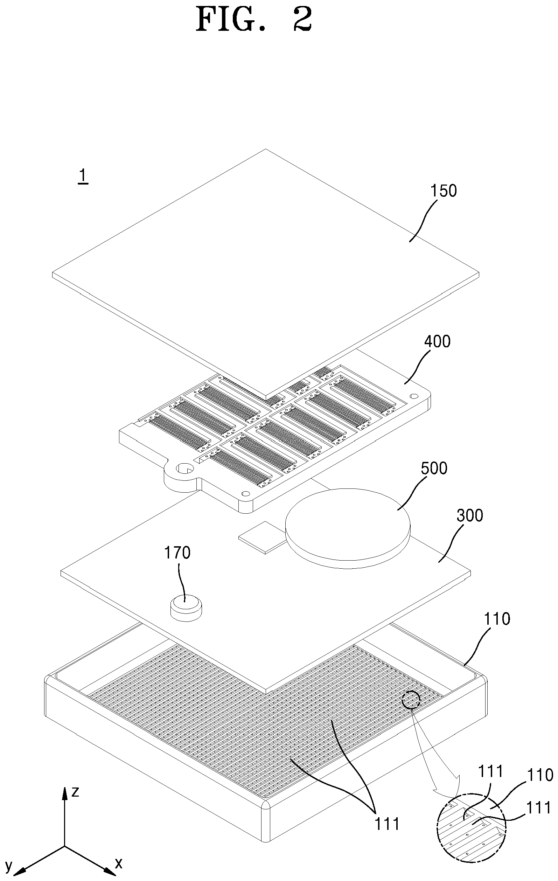

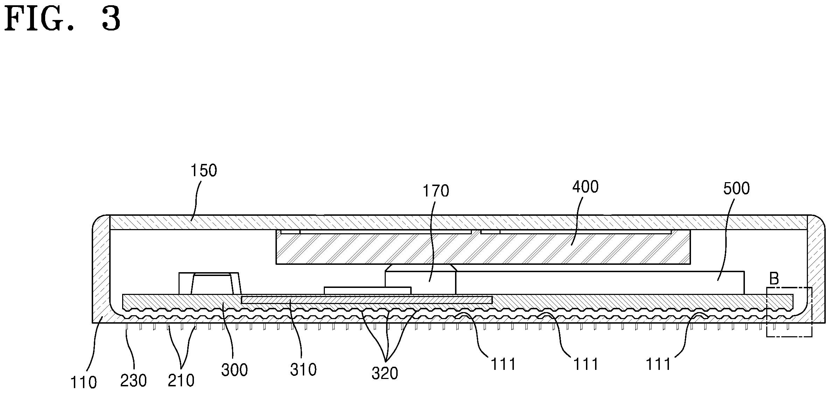

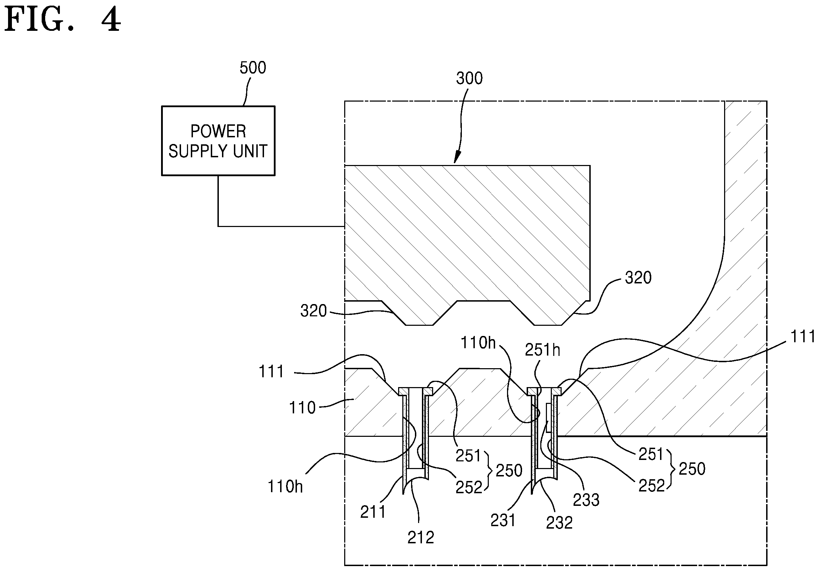

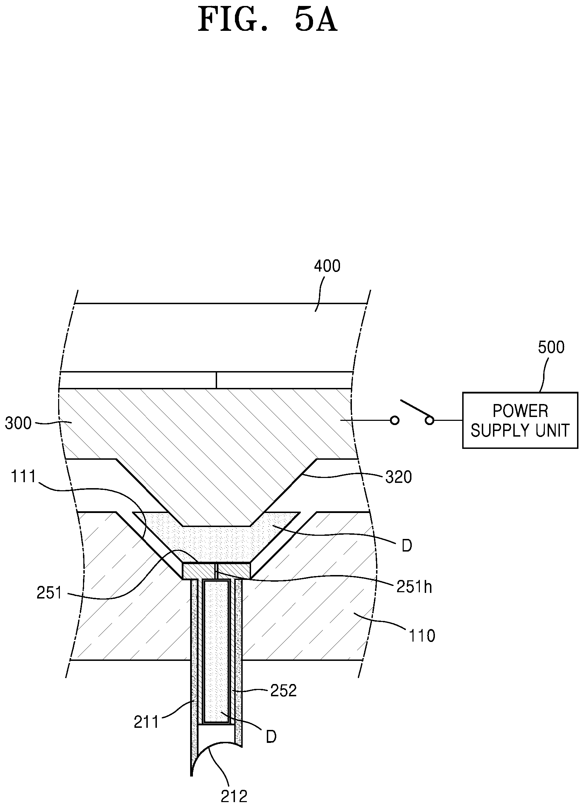

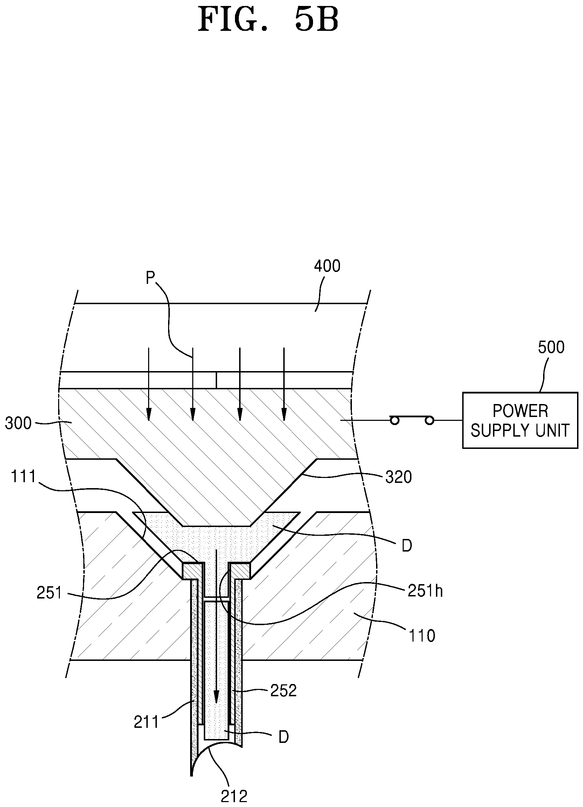

FIG. 1 is a perspective view of a medical liquid infusion apparatus according to an embodiment of the present disclosure. FIG. 2 is an exploded view of a medical liquid infusion apparatus according to an embodiment of the present disclosure. FIG. 3 is a cross-sectional view cut along a line A-A' of FIG. 1. FIG. 4 is a view schematically showing a portion B of FIG. 3. FIGS. 5A and 5B are diagrams showing use states of a medical liquid infusion apparatus according to an embodiment of the present disclosure.

A medical liquid infusion apparatus 1 may be used for various purposes according to a type of the medical liquid to be infused. For example, the medical liquid may include insulin-based medicine for diabetic patients, and other various types of medicine such as medicine for pancreas, medicine for heart, etc.

The medical liquid infusion apparatus 1 may be connected to an electronic medicine infusion control device wiredly or wirelessly. A user may control the medical liquid infusion apparatus 1 by manipulating the medicine infusion control device, and monitor a use state of the medical liquid infusion apparatus 1.

For example, the user may monitor the amount of medical liquid, the number of times medical liquid is infused, body and biometric information, etc., of the user, and control driving of the medical liquid infusion apparatus 1 based on them. For example, the medicine infusion control device may include a user terminal and will be later described in detail.

Referring to FIGS. 1 and 2, the medical liquid infusion apparatus 1 according to an embodiment of the present disclosure may include a body portion 100, a needle assembly 200, a substrate 300, a pressure generation unit 400, and a power supply unit 500.

Referring to FIGS. 1 to 4, the body portion 100 according to an embodiment of the present disclosure may form an exterior of the medical liquid infusion apparatus 1 and include a body housing 110 and a cover portion 150.

The body housing 110 according to an embodiment of the present disclosure may be connected to the needle assembly 200 described below, and accommodate the substrate 300, the pressure generation unit 400, and the power supply unit 500.

Referring to FIG. 1, the body housing 110 according to an embodiment of the present disclosure may be formed in a rectangular parallelepiped shape including an opening formed on a surface thereof.

In a selective embodiment, the body housing 110 may be formed in a cylindrical shape including an opening formed at a side thereof, but may be formed in various shapes capable of accommodating electronics, without being limited thereto.

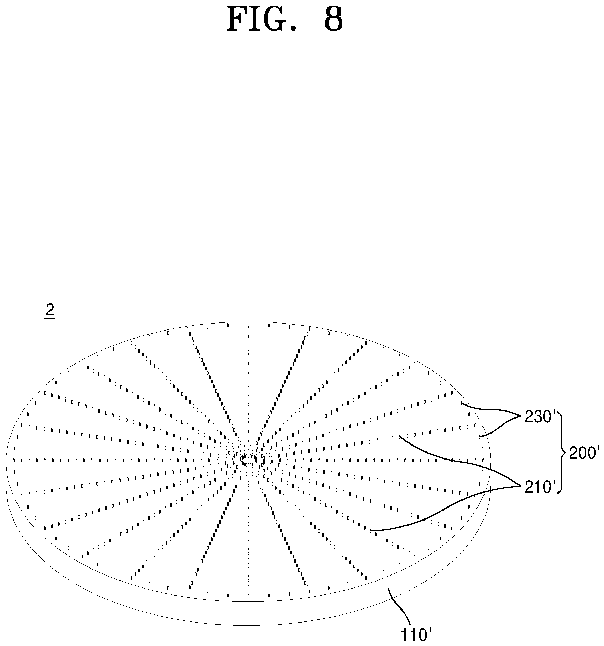

More specifically, a body housing 110' formed in a cylindrical shape is shown in FIG. 8 and will be described later.

A plurality of needle hole portions 110h may be formed in a surface of the body housing 110 contacting a user's body. Thus, an infusion needle portion 210 and a sensing needle portion 230 may pass through the needle hole portion 110h to contact and penetrate the user's body and may infuse medical liquid into the user's body.

However, various modifications may be possible without being limited to the foregoing description, such as the infusion needle portion 210, the sensing needle portion 230, and the body housing 110 being formed integrally, etc.

Referring to FIGS. 1 and 4, the plurality of needle hole portions 110h according to an embodiment of the present disclosure may be positioned in a grid shape in such a way that each needle hole portion 110h is separated from the needle hole portion 110h adjacent thereto by a preset interval on a surface of the body housing 110 contacting the user's body.

More specifically, among the plurality of needle hole portions 110h forming the grid shape, the sensing needle portion 230 may be insertedly connected to the plurality of needle hole portions 110h positioned along an outer circumference of the body housing 110 and the infusion needle portion 210 may be inserted into the other needle hole portions 110h than the needle hole portions 110h positioned along the outer circumference.

For example, when N needle hole portions 110h are formed in a direction (an x-axis direction in FIG. 1) and N needle hole portions 110h are formed in another direction (a y-axis direction in FIG. 1), the sensing needle portion 230 may be inserted into 4(N-1) needle hole portions 110h formed on an outermost side with respect to a center of a surface of the body housing 110 and the infusion needle portion 210 may be inserted into the other (N-1)² needle hole portions 110h.

Thus, as a needle groove portion 111 described below is formed by being limited to an area where the needle hole portion 110h into which the infusion needle portion 210 is inserted is formed, medical liquid D may be prevented from penetrating into the sensing needle portion 230.

In addition, the sensing needle portion 230 may effectively obtain body data from the user.

Referring to FIG. 8, in a selective embodiment, when a surface of the body housing 110' is formed in a circular shape, a needle hole portion may be formed in a radial direction with respect to the center of the surface of the body housing 110' contacting the user's body.

The plurality of needle hole portions 110h may be formed along a plurality of virtual lines equiangularly disposed with respect to the center of the surface of the body housing 110, and the sensing needle portion 230 may be inserted into each of the plurality of needle hole portions 110h formed along the virtual lines.

However, without being limited to the foregoing description, the needle hole portion 110h into which the sensing needle portion 230 is inserted and a position in the needle hole portion 110h into which the sensing needle portion 230 is inserted may be various.

Referring to FIG. 4, a diameter of the needle hole portion 110h according to an embodiment of the present disclosure may be equal to or less than an outer diameter of the infusion needle portion 210 or the sensing needle portion 230. As a result, the infusion needle portion 210 and the sensing needle portion 230 may be inserted into the needle hole portion 110h by forced fitting, thus improving an engaging force.

In a selective embodiment, a sealing member (not shown) may be arranged between the infusion needle portion 210 and the needle hole portion 110h and between the sensing needle portion 230 and the needle hole portion 110h.

Thus, the medical liquid D inside the medical liquid infusion apparatus 1 may be infused to outside through the infusion needle portion 210 and may be prevented from being discharged through the needle hole portion 110h.

Referring to FIGS. 2 and 4, the needle groove portion 111 may be formed in a groove shape inside the body housing 110 where the needle assembly 200 according to an embodiment of the present disclosure is positioned.

A thickness of an outer wall of the body housing 110 in a region where the needle groove portion 111 according to an embodiment of the present disclosure is positioned may be less than a thickness of an outer wall of the body housing 110 where the needle groove portion 111 is not positioned.

The needle groove portion 111 according to an embodiment of the present disclosure may be arranged facing a first electrode 320 formed in the shape of a protrusion and described below and may be formed in the shape of a groove corresponding to the shape of the protrusion of the first electrode 320.

As a result, the first electrode 320 may be placed on the needle groove portion 111 to contact the needle assembly 200, such that the first electrode 320 may apply voltage to the needle assembly 200, more specifically, a variable member 500.

In addition, the medical liquid D may be accommodated in the needle groove portion 111, and when the variable member 250 receives voltage and thus an opening/closing hole portion 251h described below is expanded, the medical liquid D accommodated in the needle groove portion 111 may be introduced into the needle assembly 200 through the opening/closing hole portion 251h and thus discharged to outside.

In other words, when the variable member 250 does not receive voltage, inner surfaces of the opening/closing hole portion 251h may closely contact mutually to close a space in which the medical liquid D is to flow, and when the variable member 250 receives voltage, the opening/closing hole portion 251h may be expanded and the medical liquid D may flow through an inner region of the opening/closing hole portion 251h and may be introduced into the user's body.

Referring to FIG. 2, the needle groove portion 111 according to an embodiment of the present disclosure may be formed in the shape of a groove extending in a direction (an x-axis direction in FIG. 2).

More specifically, the needle groove portion 111 may be formed in the shape of a groove extending in the direction to overlap a region where the needle hole portion 110h is formed.

As a result, the needle groove portion 111 may be formed in the shape of a groove to accommodate the medical liquid D, and when the medical liquid D accommodated in an infusion needle body 211 is exhausted, the medical liquid D accommodated in the needle groove portion 111 may be further supplemented to the infusion needle body 211.

More specifically, the first electrode 320 may apply voltage to the variable member 250 to form the opening/closing hole portion 251h, thereby communicating the needle groove portion 111 with the infusion needle body 211, and the medical liquid D accommodated in the needle groove portion 111 may be introduced into the infusion needle body 211 by the pressure generation unit 400 to supplement the medical liquid D.

Referring to FIGS. 1 and 2, the cover portion 150 according to an embodiment of the present disclosure may cover the body housing 110 and may be connected to a side of the body housing 110.

Thus, by separating the cover portion 150 from the body housing 110, electronic parts such as the needle assembly 200, the substrate 300, the pressure generation unit 400, etc., accommodated in the body housing 110, may be replaced or the medical liquid D or fluid F accommodated in the body housing 110 may be replaced or supplemented.

Moreover, by coupling the cover portion 150 to the body housing 110, the medical liquid D or the fluid F accommodated in the body housing 110 may be prevented from being discharged to outside.

Referring to FIGS. 3, 5A, and 5B, the needle assembly 200 according to an embodiment of the present disclosure may be connected to the needle hole portion 110h and contact the user's body, and may include the infusion needle portion 210, the sensing needle portion 230, and the variable member 250.

The infusion needle portion 210 according to an embodiment of the present disclosure may be capable of infusing the medical liquid D into the user's body and may include the infusion needle body 211.

The infusion needle body 211 may be formed in the shape of a hollow tube and thus accommodate the variable member 250, the medical liquid D, or the fluid F therein.

Referring to FIG. 4, the infusion needle portion 210 may be inserted into the needle hole portion 110h to communicate an inner side and an outer side of the body housing 110 with each other.

The infusion needle body 211 according to an embodiment of the present disclosure may form an exterior of the infusion needle portion 210, and an infusion hole portion 212 may be formed in the form of a hole at an end of the infusion needle body 211.

The end of the infusion needle body 211 where the infusion hole portion 212 is formed may be inserted into the user's body to infuse the medical liquid D into the user's body, and may be formed asymmetrically with respect to a longitudinal central axis.

More specifically, a side of the end may extend further in a direction (a downward direction in FIG. 4) than the other side of the end with respect to the longitudinal central axis of the infusion needle body 211 where the infusion hole portion 212 is formed.

A surface of the infusion needle body 211 where the infusion hole portion 212 is formed may be formed in the shape of a curved surface. More specifically, the surface of the infusion needle body 211 may be formed in the shape of a curved surface inclined downwardly toward the longitudinal central axis from an outer side.

As a result, the surface of the infusion needle body 211 contacting the user's body may be formed in the shape of a curved surface corresponding to the surface of the body, making it possible to reduce a user's pain when the infusion needle body 211 is inserted into the body or to help in tissue recovery when the infusion needle body 211 is removed from the user's body.

In addition, as the surface of the infusion needle body 211 contacting the user's body is formed in the shape of an ergonomically curved surface, it is possible to prevent a biological tissue of the body into which the infusion needle body 211 is inserted from being excessively cut or expanded.

In a selective embodiment, an outer circumferential surface of the infusion needle body 211 in which the infusion hole portion 212 is formed may be formed to be inclined downwardly toward the longitudinal central axis toward the end where the infusion hole portion 212 is formed.

As a result, when the end of the infusion needle body 211 is inserted into the body, the end may be inserted closely to the longitudinal central axis, thereby preventing the tissue in the body from being excessively cut or expanded.

An outer diameter of the infusion needle body 211 according to an embodiment of the present disclosure may be equal to or greater than an inner diameter of the needle hole portion 110h. As a result, the infusion needle portion 210 and the sensing needle portion 230 may be coupled to the needle hole portion 110h by forced fitting.

The infusion needle body 211 according to an embodiment of the present disclosure may be formed as a non-conductor. Thus, when voltage is applied from the first electrode 320 to the variable member 250, current may be prevented from flowing to the infusion needle body 211, thereby preventing the current from flowing through the user's body connected to the infusion needle body 211.

Referring to FIG. 4, an inner diameter of the infusion needle body 211 may be equal to or less than an outer diameter of the variable member 250, more specifically, an accommodation portion 252 described below. Thus, the variable member 250 may be fixed by forcedly fitting into the infusion needle body 211.

Referring to FIG. 4, the inner diameter of the infusion needle body 211 may be less than an outer diameter of an opening/closing portion 251.

As a result, the opening/closing portion 251 may contact an end (an upper end in FIG. 4) of the infusion needle body 211 and thus movement of the variable member 250 in a direction (the downward direction in FIG. 4) may be limited, thereby preventing the variable member 250 from penetrating the infusion needle body 211 and moving outside even when the pressure generation unit 400 applies pressure to the medical liquid D accommodated in the needle groove portion 111 or the infusion needle body 211.

Referring to FIG. 4, the sensing needle portion 230 according to an embodiment of the present disclosure may collect body data of the user and include a sensing needle body 231 and a sensor unit 233.

In a selective embodiment, the sensing needle portion 230 may measure a user's blood sugar level using a blood-gathering blood sugar level measurement method. More specifically, the sensing needle portion 230 may measure the blood sugar level of the user by using an electrochemical oxidation-reduction reaction between glucose oxidase enzyme and glucose.

Referring to FIG. 4, the sensing needle body 231 according to an embodiment of the present disclosure may form an exterior of the sensing needle portion 230 and may be formed in the shape of a hollow tube.

Thus, the variable member 250, a substance including user's body data, or the fluid F may be accommodated in the sensing needle body 231.

Referring to FIG. 4, the sensing needle portion 230 may be inserted into the needle hole portion 110h to communicate the inner side and the outer side of the body housing 110 with each other.

A sensing hole portion 232 may be formed in the shape of a hole at an end of the sensing needle body 231 according to an embodiment of the present disclosure. Thus, the substance including the user's body data may move into the medical liquid infusion apparatus 1 through the sensing hole portion 232.

The end of the sensing needle body 231 where the sensing hole portion 232 is formed may be inserted into the user's body to obtain the substance including the user's body data, and may be formed asymmetrically with respect to a longitudinal central axis.

More specifically, a side of the end may extend further in a direction (a downward direction in FIG. 4) than the other side of the end with respect to the longitudinal central axis of the sensing needle body 231 where the sensing hole portion 232 is formed.

A surface of the sensing needle body 231 where the sensing hole portion 232 is formed may be formed in the shape of a curved surface. More specifically, the surface of the sensing needle body 231 may be formed in the shape of a curved surface inclined downwardly toward the longitudinal central axis from an outer side.

As a result, the surface of the sensing needle body 231 contacting the user's body may be formed in the shape of a curved surface corresponding to the surface of the body, making it possible to reduce a user's pain when the sensing needle body 231 is inserted into the body, or to help in tissue recovery when the sensing needle body 231 is removed from the user's body.

In addition, as the surface of the sensing needle body 231 contacting the user's body is formed in the shape of an ergonomically curved surface, it is possible to prevent a biological tissue of the body into which the sensing needle body 231 is inserted from being excessively cut or expanded.

In a selective embodiment, an outer circumferential surface of the sensing needle body 231 in which the sensing hole portion 232 is formed may be formed to be inclined downwardly toward the longitudinal central axis toward the end where the sensing hole portion 232 is formed.

As a result, when the end of the sensing needle body 231 is inserted into the body, the end may be inserted closely to the longitudinal central axis, thereby preventing the tissue in the body from being excessively cut or expanded.

An outer diameter of the sensing needle body 231 according to an embodiment of the present disclosure may be equal to or greater than the inner diameter of the needle hole portion 110h. Thus, the sensing needle portion 230 may be coupled to the needle hole portion 110h by forced fitting.

The sensing needle body 231 according to an embodiment of the present disclosure may be formed as a non-conductor. Thus, when voltage is applied from the first electrode 320 to the variable member 250, current may be prevented from flowing to the sensing needle body 231, thereby preventing the current from flowing through the user's body connected to the sensing needle body 231.

Referring to FIG. 4, the inner diameter of the sensing needle body 231 may be equal to or less than the outer diameter of the variable member 250, more specifically, the accommodation portion 252 described below. Thus, the variable member 250 may be fixed by forcedly fitting into the sensing needle body 231.

Referring to FIG. 4, the sensor unit 233 according to an embodiment of the present disclosure may extract body data from a body substance of the user in contact with the body substance.

Herein, the 'body substance' may include, but not limited to, blood, cell tissue, etc., of the user, and any substance from which the user's body data may be extracted.

The sensor unit 233 according to an embodiment of the present disclosure may be positioned in the sensing needle body 231, without being limited thereto, and may be disposed in various positions enabling contact with a substance including the user's body data, such as an outside of the sensing needle body 231, etc.

The sensor unit 233 according to an embodiment of the present disclosure may receive voltage from the first electrode 320 and generate current. The sensor unit 233 may allow current to flow by using the body substance obtained from the sensing hole portion 232 as an electrode, and measure a user's blood sugar level by using a difference between a strength of initial current and a strength of current based on the body substance used as the electrode.

However, the present disclosure is not limited thereto, and a method for the sensor unit 233 to measure the user's blood sugar level may include any conventional blood sugar level measurement method.

Referring to FIGS. 4, 5A, and 5B, the variable member 250 according to an embodiment of the present disclosure may receive voltage from the substrate 300 described below so as to be shape-deformed, and may be placed at sides of the infusion needle portion 210 and the sensing needle portion 230.

Referring to FIG. 4, the variable member 250 according to an embodiment of the present disclosure may include the opening/closing portion 251 that may contact the substrate 300 and receive voltage from the substrate 300 to form the opening/closing hole portion 251h, and the accommodation portion 252 capable of accommodating the medical liquid D, etc.

The variable member 250 according to an embodiment of the present disclosure may include a conductive polymer material. For example, the variable member 250 may include a combination of poly 3,4-enthlenedioxythiophene (PEDOT:PSS) and multiwall carbon nanotubes.

However, without being limited to the foregoing description, the variable member 250 may be formed of various materials within a technical spirit in which the variable member 250 is shape-deformable by receiving voltage.

The opening/closing portion 251 according to an embodiment of the present disclosure may be formed at the end (the upper end in FIG. 4) of the variable member 250, and may be positioned to contact the first electrode 320.

More specifically, the opening/closing portion 251 may contact the first electrode 320 to receive voltage from the first electrode 320 and thus may be shape-deformed, and may form, more specifically, the opening/closing hole portion 251h.

Referring to FIGS. 4 and 5B, the opening/closing hole portion 251h according to an embodiment of the present disclosure may be formed in the shape of a hole that communicates an inner space and an outer space of the sensing needle portion 230 or the infusion needle portion 210 with each other, and may share the longitudinal central axis with the variable member 250.

However, without being limited to the foregoing description, the opening/closing hole portion 251h may be formed in various shapes within a technical spirit in which the opening/closing hole portion 251h communicates the inner space and the outer space of the sensing needle portion 230 or the infusion needle portion 210 with each other.

Referring to FIGS. 4 and 5, the accommodation portion 252 according to an embodiment of the present disclosure may accommodate the medical liquid D, the fluid F, or the fluid F including the body data, and may be formed in the shape of a hollow tube.

Hereinbelow, a description will be made of the shapes of the opening/closing portion 251 respectively corresponding to a case where the variable member 250 receives voltage from the first electrode 320 and a case where the variable member 250 does not receive the voltage.

Referring to FIG. 5A, when the opening/closing portion 251 does not receive the voltage from the first electrode 320, the opening/closing portion 251 may form a wall to separate an inner space and an outer space of the accommodation portion 252.

Thus, even when the pressure generation unit 400 applies pressure to the medical liquid D or the fluid F accommodated in the needle groove portion 111, the medical liquid D or the fluid F pressed by the opening/closing portion 251 forming the wall may be prevented from being introduced to the inner space of the accommodation portion 252, thus controlling infusion of the medical liquid D into the body.

Referring to FIG. 5B, when the opening/closing portion 251 receives the voltage from the first electrode 320, the opening/closing portion 251 may form the opening/closing hole portion 251h that communicates the inner space and the outer space of the accommodation portion 252 with each other.

As a result, the medical liquid D accommodated in the needle groove portion 111 may be introduced to the inner space of the accommodation portion 252 through the opening/closing hole portion 251h, such that the pressure generation unit 400 may press the medical liquid D accommodated in the needle groove portion 111 to discharge the medical liquid D into the body through the infusion hole portion 212.

As such, the medical liquid infusion apparatus 1 according to an embodiment of the present disclosure may block or allow infusion of the medical liquid D by using voltage instead of adjusting the amount of medical liquid D to be infused into the body by using a driving device such as a piston, etc., thereby precisely controlling an ultra-small amount of medical liquid to be infused.

The opening/closing portion 251 and the accommodation portion 252 according to an embodiment of the present disclosure may be formed integrally, but without being limited thereto, may also be formed as separate pieces and of different materials.

In a selective embodiment, the opening/closing portion 251 may be formed of a conductive material, and the accommodation portion 252 may be formed of a nonconductive material.

As a result, when the first electrode 320 applies voltage to the opening/closing portion 251, the shape of the opening/closing portion 251 may be deformed.

Referring to FIGS. 2 to 4, the substrate 300 according to an embodiment of the present disclosure may apply voltage to the needle assembly 200 and may include a processor 310 and the first electrode 320.

The processor 310 according to an embodiment of the present disclosure may be implemented using at least one of application specific integrated circuits (ASICs), digital signal processors (DSPs), digital signal processing devices (DSPDs), programmable logic devices (PLDs), field programmable gate arrays (FPGAs), controllers, microcontrollers, microprocessors, and other electric units for performing functions.

The processor 310 according to an embodiment of the present disclosure may include a volatile or non-volatile memory that may store a command or data related to at least one other component of the medical liquid infusion apparatus 1.

The processor 310 may store software or a program. A program may include, for example, a kernel, middleware, an application programming interface (API), an application program (or "application"), etc.

At least a part of the kernel, the middleware, or the API may be referred to as an operating system. The kernel, for example, may control or manage system resources (e.g., a bus, a processor, or memory, etc.) used to execute operations or functions implemented in other programs (e.g., the middleware, the API, or the application program).

In addition, the kernel may provide an interface to control or manage system resources by accessing individual components of the medical liquid infusion apparatus 1 in the middleware, the API, or the application program.

The processor 310 according to an embodiment of the present disclosure may receive body data from the sensing needle portion 230 and generate a control signal.

The control signal according to an embodiment of the present disclosure may be a wired signal or a wireless signal.

When the control signal is implemented as a wired signal, the control signal may include, but not limited to, at least one of a universal serial bus (USB), a high definition multimedia interface (HDMI), a recommended standard 232 (RS-232), power line communication, a plain old telephone service (POTS), etc., and may be transmitted or received in various ways within a technical spirit in which an electric signal may be transmitted using a wired device.

When the control signal is implemented as a wireless signal, the control signal may include at least one of Bluetooth, Bluetooth Low Energy (BLE), Wireless Fidelity (WiFi), ZigBee, near field communication (NFC), magnetic secure transmission, radio frequency (RF), or a body area network (BAN).

The control signal implemented as a wireless signal may include cellular communication using at least one of, for example, long-term evolution (LTE), LTE advance (LTE-A), code division multiple access (CDMA), wideband CDMA (WCDMA), universal mobile telecommunications system (UMTS), wireless broadband (WiBro), global system for mobile communications (GSM), etc.

However, the present disclosure is not limited thereto, and the control signal may include a global navigation satellite system (GNSS). The GNSS may include, for example, at least one of a global positioning system (GPS), a global navigation satellite system (Glonass), a Beidou navigation satellite system ("Beidou"), or Galileo, the European global satellite-based navigation system.

The processor 310 may selectively apply voltage to the variable member 250 through the first electrode 320 by transmitting the control signal to the first electrode 320.

The processor 310 according to an embodiment of the present disclosure may selectively apply voltage to the variable member 250 according to body data obtained from the sensing needle portion 230 or a command obtained from an external user terminal, etc.

As a result, the medical liquid D may be discharged according to the voltage selectively applied by the first electrode 320 based on the control signal generated by the processor 310, such that the processor 310 may control the amount, an infusion period, an infusion time, etc., of the medical liquid D to be infused to the user according to the body data or the external command.

Referring to FIG. 3, the first electrode 320 according to an embodiment of the present disclosure may apply voltage to the variable member 250 and may be positioned at a side of the substrate 300 facing the needle assembly 200.

Referring to FIG. 4, the variable member 250 may be formed in the shape of a protrusion extending toward the variable member 250 from a surface of the substrate 300, which faces the needle assembly 200. More specifically, the shape of the protrusion of the variable member 250 may be formed to correspond to the shape of the needle groove portion 111.

Thus, the first electrode 320 according to an embodiment of the present disclosure may be placed on the needle groove portion 111, and more specifically, the first electrode 320 may be placed on the needle groove portion 111 to contact the variable member 250.

In a selective embodiment, the first electrode 320 may receive external voltage to move in a vertical direction (in FIG. 4). More specifically, the first electrode 320 may receive a control signal of the processor 310 and move in the vertical direction according to the control signal.

Thus, as the first electrode 320 moves in a downward direction to contact the variable member 250, the voltage may be applied to the variable member 250, and as the first electrode 320 moves in the upward direction to be spaced apart from the variable member 250, the voltage applied to the variable member 250 may be removed.

In addition, as a space between the first electrode 320 and the needle groove portion 111 may be reduced by downward movement of the first electrode 320, pressure may be applied to the medical liquid D accommodated in the needle groove portion 111, and as the space between the first electrode 320 and the needle groove portion 111 may be expanded by upward movement of the first electrode 320, the pressure applied to the medical liquid D accommodated in the needle groove portion 111 may be removed.

Referring to FIGS. 2 and 3, the pressure generation unit 400 according to an embodiment of the present disclosure may generate and provide pressure P to discharge the medical liquid D to outside.

The pressure generation unit 400 according to an embodiment of the present disclosure may have a preset weight, and the substrate 300 may be provided with the pressure P in the downward direction (in FIG. 3) by the weight of the pressure generation unit 400.

Thus, the first electrode 320 of the substrate 300 may apply the pressure P to the medical liquid D accommodated in the needle groove portion 111, and the medical liquid D may receive the pressure P and may be infused into the user's body through the needle hole portion 110h.

The pressure generation unit 400 according to an embodiment of the present disclosure may apply pressure to the substrate 300 at all times, and the medical liquid D receiving the pressure at all times by the substrate 300 may be maintained in a pressed state.

Thus, when the first electrode 320 applies voltage to the variable member 250 based on the control signal generated by the processor 310, the variable member 250 may form the opening/closing hole portion 251h and the pressed medical liquid D may be infused into the user's body through the opening/closing hole portion 251h and the needle hole portion 110h.

Referring to FIG. 3, the pressure generation unit 400 may be connected to an inner side of a cap portion and may be connected to the substrate 300 through a connection member 170.

More specifically, a surface of the pressure generation unit 400 may closely contact a surface of the cap portion, and the other surface of the pressure generation unit 400 may closely contact a surface of the substrate 300 directly, in which the connection member 170 may be disposed between the pressure generation unit 400 and the substrate 300 by forced fitting.

For example, a sum of a length of the pressure generation unit 400 in a direction (the upward direction in FIG. 3), a length of the connection member 170 in the direction, and a length of the substrate 300 in the direction may be greater than or equal to a length of an inner space of the body housing 110 in the direction.

Thus, the cap portion, the pressure generation unit 400, the connection member 170, and the substrate 300 may closely contact one another inside the body housing 110, such that there may be a normal force of a preset magnitude on a contact interface and the substrate 300 may apply pressure to the medical liquid D due to the normal force.

In a selective embodiment, the pressure generation unit 400 may be position-changed by a driving device (not shown).

More specifically, the pressure generation unit 400 may be connected to a side of the body portion 100 through a driving device such as an actuator, a camshaft, etc., and the pressure generation unit 400 may move in the upward direction (in FIG. 3) through the driving device.

The driving device may drive the pressure generation unit 400 to move in the downward direction according to the control signal of the processor 310, and the pressure generation unit 400 may apply pressure to the substrate 300 while moving in the downward direction.

Thus, the medical liquid D that may contact the first electrode 320 of the substrate 300 may receive the pressure and may be infused into the user's body through the needle groove portion 111.

The driving device may drive the pressure generation unit 400 to move in the upward direction according to the control signal of the processor 310, and the pressure generation unit 400 may remove the pressure applied to the substrate 300 while moving in the upward direction.

Thus, as the pressure applied to the medical liquid D that may contact the first electrode 320 of the substrate 300 is removed, the medical liquid D may undergo a decompression process, thus limiting infusion of the medical liquid D into the user's body.

In a selective embodiment, the pressure generation unit 400 may be disposed to directly contact the medical liquid D. As a result, the pressure generation unit 400 may directly apply the pressure to the medical liquid D without passing through the substrate 300.

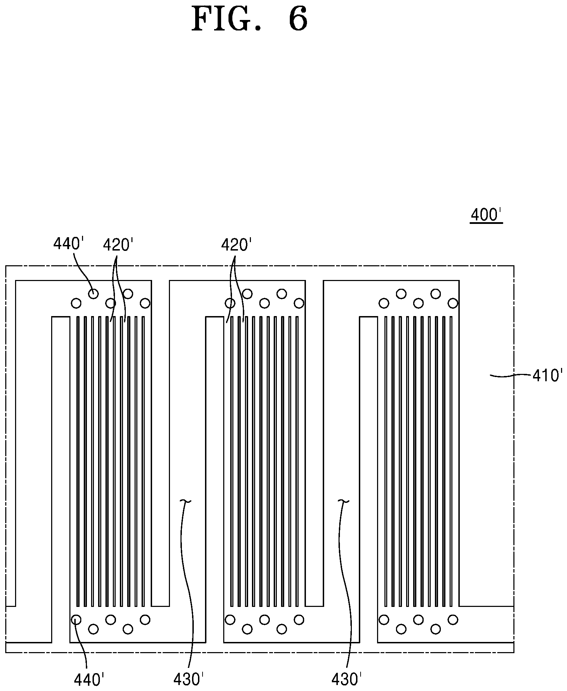

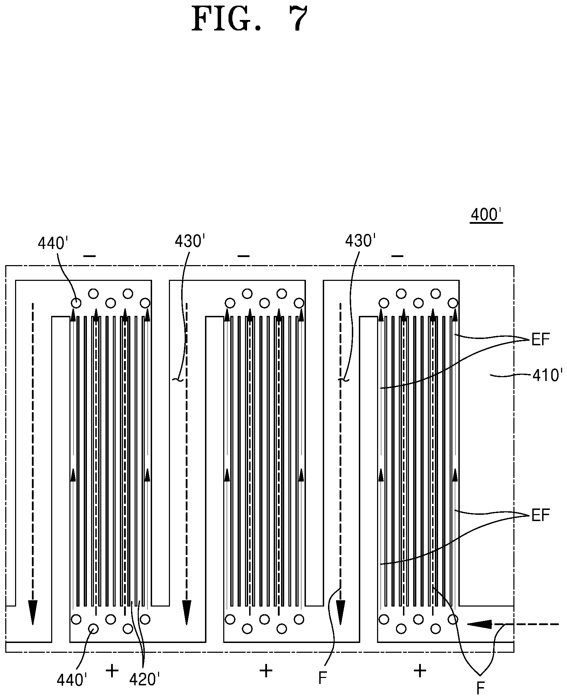

FIG. 6 is a plan view of a pressure generation unit according to another embodiment of the present disclosure. FIG. 7 is a view showing flow of fluid in a pressure generation unit according to another embodiment of the present disclosure.

Referring to FIGS. 6 and 7, a pressure generation unit 400' according to another embodiment of the present disclosure may provide pressure to the medical liquid D by using pressure of the fluid F and include a pressing housing 410', a first flow path 420', a second flow path 430', and a second electrode 440'.

The pressing housing 410' may form an exterior of the pressure generation unit 400' and may be positioned in an inner side of the body portion.

The first flow path 420' and the second flow path 430' may be formed at a side of the pressing housing 410' in the form of a groove, but without being limited thereto, the first flow path 420' and the second flow path 430' may be formed separately from the pressing housing 410' and in a shape connected to the pressing housing 410'.

Referring to FIG. 6, the first flow path 420' may allow the fluid F to flow therethrough and may be formed on a surface of the pressing housing 410' in the shape of a groove extending in the vertical direction (in FIG. 6).

A plurality of first flow paths 420' may be provided, and opposite ends thereof may communicate with one second flow path 430'.

Referring to FIGS. 6 and 7, the second flow path 430' formed in the pressure generation unit 400' may communicate with the first flow path 420' and provide a flow path for the fluid F. A plurality of second flow paths 430' may be provided and may be disposed respectively in inlets and outlets of the first flow paths 420'.

**In** a selective embodiment, a sum of cross-sectional areas of the plurality of first flow paths 420' may be greater than a cross-sectional area of one second flow path 430'.

More specifically, A1 or n may be set to satisfy A1 X n > A2 when a cross-sectional area of the first flow path 420' is A1, a cross-sectional area of the second flow path 430' is A2, and the number of first flow paths 420' communicating with one second flow path 430' is n.

Thus, as a total area of a flow path through which a unit mass flow rate of the fluid F flows increases from the cross-sectional area of one second flow path 430' to a sum of the cross-sectional areas of the plurality of first flow paths 420', the fluid F may undergo a pressurizing process according to the Bernoulli's law.

Referring to FIGS. 6 and 7, the second electrode 440' may form an electric field EF at a side of the pressure generation unit 400' and may be positioned at opposite ends of the first flow path 420'.

Referring to FIG. 7, the second electrode 440' may include a positive electrode and a negative electrode. More specifically, the positive electrode may be disposed at an end of the first flow path 420' through which the fluid F is introduced, and the negative electrode may be disposed at the other end of the first flow path 420' through which the fluid F is discharged to the second flow path 430'.

Thus, when the second electrode 440' receives voltage from a power supply unit, an electric field may be formed on the first flow path 420' and the fluid F accommodated in the first flow path 420' may flow in a direction of the electric field, i.e., from the positive electrode to the negative electrode.

As a result, the fluid F accommodated in the pressure generation unit 400' may not move until the second electrode 440' receives voltage from the power supply unit, and the fluid F accommodated in the first flow path 420' may have a preset speed when the electric field is formed after the second electrode 440' receives the voltage, such that corresponding pressure is generated and the fluid F may provide pressure for discharging the medical liquid D.

Moreover, when a sum of the cross-sectional areas of the plurality of first flow paths 420' is greater than the cross-sectional area of one second flow path 430', then the electric field EF may be formed in a direction from bottom to top (in FIG. 7) such that the direction of the electric field EF matches the flow direction of the fluid F in the first flow path 420', and thus a flow rate of the fluid F may increase in the first flow path 420' due to the electric field EF.

Thus, as a total area of a flow path through which the unit mass flow rate of the fluid F flows increases from the cross-sectional area of one second flow path 430' to the sum of the cross-sectional areas of the plurality of first flow paths 420', the flow rate of the fluid F in the first flow path 420' may decrease according to the Bernoulli's law.

Referring to FIGS. 6 and 7, the fluid F accommodated in the second flow path 430' may not flow due to the electric field formed by the second electrode 440' receiving voltage from the power supply unit, like the first flow path 420', but due to pressure generated in the first flow path 420', the fluid F may flow to the first flow path 420' at the next step.

More specifically, the plurality of first flow paths 420' and the plurality of second flow paths 430' may be provided, and a process in which the fluid F passing through the first flow path 420' having the second electrode 440' disposed at opposite sides thereof enters the second flow path 430' may be referred to as a 'single step'.

The plurality of first flow paths 420' and the plurality of second flow paths 430' may be sequentially disposed, and a plurality of single steps may be connected to form "multiple steps'.

Pressure may be generated in the fluid F while the fluid F passes through the first flow path 420' of the single step, and the pressure may increase as the fluid F passing through the second flow path 430' passes through the first flow path 420' of another single step.

The pressure generation unit 400' according to another embodiment of the present disclosure may generate pressure targeted by the pressure generation unit 400' according to the number of single steps of multiple steps.

In a selective embodiment, the second electrode 440' may be positioned at opposite ends of the second flow path 430'. In this case, the negative electrode may be positioned at an end of the second flow path 430' through which the fluid F is introduced, and the positive electrode may be positioned at the other end of the second flow path 430' through which the fluid F is discharged to the second flow path 430'.

As a result, the electric field **EF** matches the flow direction of the fluid F flowing through the second flow path 430' and thus may apply acceleration and pressure to the fluid F.

The fluid F pressed by passing through the first flow path 420' and the second flow path 430' may apply pressure to the medical liquid D placed on the needle groove portion 111 and the needle assembly.

More specifically, the pressing housing 410' and the plurality of needle groove portions 111 may be connected through separate tubes, and the pressed fluid F may flow from the pressing housing 410' to the needle groove portion 111 through the tube, thus pushing the medical liquid D placed in the needle groove portion 111 to the infusion hole portion 212.

However, the present disclosure is not limited thereto, and the pressing fluid F discharged from the pressing housing 410' may fill an inner space of the body housing 110 including a space between the substrate 300 and the needle hole portion 110h.

Thus, the inner space of the body housing 110 may be formed at a high pressure due to the pressing fluid **F,** such that when the variable member 250 receives voltage from the substrate 300, the opening/closing hole portion 251h may be expanded and thus the medical liquid D may be discharged from an inner side of the body housing 110, more specifically, the needle hole portion 110h, to the infusion hole portion 212.

Referring to FIGS. 2 and 3, the power supply unit 500 according to an embodiment of the present disclosure may apply voltage to electronic devices accommodated in the medical liquid infusion apparatus 1, such as the substrate 300, the pressure generation unit 400, etc.

The power supply unit 500 according to an embodiment of the present disclosure may be electrically connected to the substrate 300 and the pressure generation unit 400, and may supply voltage to the substrate 300 and the pressure generation unit 400.

A detailed structure of the power supply unit 500 is well known and thus will not be described in detail.

Hereinbelow, operating principles and effects of the medical liquid infusion apparatus 1 according to an embodiment of the present disclosure will be described.

Herein, describing the operating principles and the effects in time series is for the convenience of description, and the operating principles described below are not limited to the order of description and may be made at the same time.

Referring to FIG. 1, the medical liquid infusion apparatus 1 according to an embodiment of the present disclosure may be connected to a part of the body of the user to infuse the medical liquid D accommodated in the medical liquid infusion apparatus 1 to the user.

The body portion 100 according to an embodiment of the present disclosure may form the exterior of the medical liquid infusion apparatus 1, the needle assembly 200 may be connected to a side of the body portion 100, and the substrate 300, the power generation unit 400, and the power supply unit 500 may be received inside the body portion 100.

The body portion 100 according to an embodiment of the present disclosure may form a square tube shape.

Referring to FIG. 1, the needle assembly 200 may be connected to a surface (a surface parallel to an xy plane in FIG. 1) of the body portion100 according to an embodiment of the present disclosure, which contacts the user.

Thus, the surface of the body portion 100 may contact the user's body and at the same time, the needle assembly 200 connected to the surface may be inserted into the user's body, and the medical liquid D accommodated in the body portion 100 or the needle assembly 200 may be infused into the user's body through the needle assembly 200.

Referring to FIG. 2, the plurality of needle groove portions 111 may be formed in the shape of a groove inside the body portion 100 according to an embodiment of the present disclosure.

More specifically, the plurality of needle groove portions 111 may be formed in the shape of a groove extending in a direction (the x-axis direction in FIG. 2) or another direction (the y-axis direction in FIG. 2) on an inner side of the body portion 100 corresponding to the surface of the body portion 100 to which the needle assembly 200 is connected. Thus, the medical liquid D may be accommodated in the plurality of needle groove portions 111.

Referring to FIGS. 2 and 4, the infusion hole portion 212 may be formed on an inner circumferential surface of the needle groove portion 111 according to an embodiment of the present disclosure, such that the needle groove portion 111 may communicate with the infusion needle portion 210 or the sensing needle portion 230 through the infusion hole portion 212.

Thus, the medical liquid D accommodated in the needle groove portion 111 may receive pressure from the pressure generation unit 400, etc., and flow to the infusion needle portion 210 through the infusion hole portion 212.

Referring to FIGS. 3 and 4, the pressure generation unit 400 positioned at a side (an upper side in FIG. 4) of the needle groove portion 111 may indirectly apply pressure to the medical liquid D accommodated in the needle groove portion 111 through the substrate 300, such that the medical liquid D accommodated in the needle groove portion 111 may receive pressure in the downward direction (in FIG. 4) to flow to the infusion needle portion 210 through the infusion hole portion 212.

However, the present disclosure is not limited thereto, such that a method of applying pressure to the needle groove portion 111 from the pressure generation unit 400 may be implemented in various ways such as a way in which the pressure generation unit 400 is directly connected to the needle groove portion 111 to apply pressure, etc.

Referring to FIGS. 2 to 4, the needle assembly 200 according to an embodiment of the present disclosure may include the infusion needle portion 210, the sensing needle portion 230, and the variable member 250.

Referring to FIG. 4, the infusion needle portion 210 and the sensing needle portion 230 according to an embodiment of the present disclosure may accommodate the variable member 250.

As the variable member 250 may be shape-deformed by application of voltage, the variable member 250 may communicate the needle groove portion 111 with the inner space of the infusion needle portion 210 or the needle groove portion 111 with the inner space of the sensing needle portion 230.

The opening/closing portion 251 positioned in the upper side (in FIG. 4) of the variable member 250 according to an embodiment of the present disclosure may protrude by a preset height from a surface of the needle groove portion 111 where the needle hole portion 110h is formed.

Thus, as the substrate 300 is placed on the needle groove portion 111, the substrate 300 may contact the opening/closing portion 251, such that the first electrode 320 of the substrate 300 may apply voltage to the opening/closing portion 251 of the variable member 250.

Hereinbelow, a process, performed by the substrate 300, of applying voltage to the variable member 250 and a shape of the variable member 250 upon application of the voltage will be described in detail.

Referring to FIGS. 3 and 4, the first electrode 320 capable of applying voltage may be formed on a surface of the substrate 300 facing the variable member 250.

The first electrode 320 according to an embodiment of the present disclosure may be placed on the needle groove portion 111 and may be position-fixed to contact the variable member 250.

The processor 310 of the substrate 300 may generate a control signal from a command received from outside or body data obtained from the sensing needle portion 230, and control the first electrode 320 to selectively apply voltage to the variable member 250 according to the control signal.

An algorithm, executed by the processor 310, to generate the control signal from the body data will be described later in detail.

The first electrode 320 may apply voltage the variable member 250, more specifically, the opening/closing portion 251, according to the control signal.

The opening/closing portion 251 may be formed of a material with a shape changing upon application of the voltage thereto. For example, the opening/closing portion 251 may be formed of a conductive polymer material or a shape-memory alloy.

Referring to FIGS. 4 and 5A, when the opening/closing portion 251 does not receive the voltage from the first electrode 320, the opening/closing portion 251 may form a barrier to separate an inner space of the needle groove portion 111 from an inner space of the infusion needle portion 210.

When the processor 310 generates a control signal to prevent the first electrode 320 from applying the voltage to the opening/closing portion 251, the opening/closing portion 251 not having received the voltage may form the barrier.

As a result, the medical liquid D accommodated in the needle groove portion 111 may be prevented by the barrier from flowing to the inner space of the infusion needle portion 210, thus stopping infusing the medical liquid D to the user.

Referring to FIGS. 4 and 5B, when the opening/closing portion 251 receives the voltage from the first electrode 320, the opening/closing portion 251 may form the opening/closing hole portion 251h to communicate the inner space of the needle groove portion 111 with the inner space of the infusion needle portion 210.

Thus, when the processor 310 generates a control signal to cause the first electrode 320 to apply the voltage to the opening/closing portion 251, the opening/closing portion 251 having received the voltage may form the opening/closing hole portion 251h.

The medical liquid D accommodated in the needle groove portion 111 may flow to the inner space of the infusion needle portion 210 through the needle hole portion 110h, and the medical liquid D accommodated in the infusion needle portion 210 may be infused to the user through the infusion hole portion 212.

In a selective embodiment, the substrate 300 may move in the vertical direction (in FIG. 4).

More specifically, the voltage may be applied to the first electrode 320 at all times, and the processor 310 may generate the control signal from the command received from an outside or the body data obtained from the sensing needle portion 230, and move the substrate 300 downwardly to contact the opening/closing portion 251 or move the substrate 300 upwardly to be separated from the opening/closing portion 251 according to the control signal.

Thus, the first electrode 320 of the substrate 300 moving downwardly according to the control signal of the processor 310 may contact the opening/closing portion 251 to apply the voltage to the opening/closing portion 251, such that the opening/closing portion 251 having received the voltage may form the opening/closing hole portion 251h to allow the medical liquid D accommodated in the needle groove portion 111 to flow to the infusion needle portion 210.

In addition, the first electrode 320 of the substrate 300 moving upwardly according to the control signal of the processor 310 may be separated from the opening/closing portion 251 to remove the voltage applied to the opening/closing portion 251, such that the opening/closing portion 251 having received the voltage may form the barrier to restrict the medical liquid D accommodated in the needle groove portion 111 from flowing to the infusion needle portion 210.

As such, the medical liquid infusion apparatus 1 may allow or block infusion of the medical liquid D in a manner that the first electrode 320 applies the voltage to the variable member 250 or removes the voltage applied thereto.

Thus, while a conventional medical liquid infusion apparatus may not be able to precisely control an infusion amount of the medical liquid D by adjusting the amount of the medical liquid D infused into the body using a driving device such as a piston, etc., a small infusion amount of the medical liquid D may be precisely controlled by controlling infusion of the medical liquid D using voltage.

Moreover, unlike a conventional medical liquid infusion apparatus that needs to generate a driving force for driving a driving device such as a piston, etc., the present disclosure may control infusion of medical liquid by applying or removing voltage, thereby reducing required power consumption.

Hereinbelow, an algorithm, executed by the processor 310, to generate the control signal from the body data will be described later in detail.

The processor 310 may be provided with the body data from the sensor unit 233 of the sensing needle portion 230.

The sensor unit 233 may measure the body data from a body substance obtained from the sensing needle portion 230.

A method for the sensor unit 233 to measure the body data from the body substance may be applied variously to a conventional body data measurement device, and thus an internal structure and operating principles of the sensor unit 233 will not be described in detail.

Herein, the body data may include, but not limited to, a user's blood sugar level, a glycated hemoglobin level, etc.

When the body data measured in the sensing needle portion 230 exceeds a preset value, the processor 310 may generate the control signal to control the first electrode 320 to apply the voltage to the variable member 250.

For example, when the user's sugar level measured in the sensing needle portion 230 exceeds a preset value, the processor 310 may generate the control signal to control the first electrode 320 to apply the voltage to the variable member 250.

As a result, the opening/closing portion 251 of the variable member 250 having received the voltage from the first electrode 320 may form the opening/closing hole portion 251h such that the medical liquid D accommodated in the needle groove portion 111 may be infused to the user's body.

When the body data measured in the sensing needle portion 230 does not exceed the preset value, the processor 310 may generate the control signal to control the first electrode 320 not to apply the voltage to the variable member 250.

For example, when the user's sugar level measured in the sensing needle portion 230 is less than the preset value, the processor 310 may generate the control signal to control the first electrode 320 not to apply the voltage to the variable member 250.

As a result, the opening/closing portion 251 of the variable member 250 having not received the voltage may form a barrier, thus blocking the medical liquid D accommodated in the needle groove portion 111 from being infused into the user's body through the infusion needle portion 210.

**In** a selective embodiment, the processor 310 may generate the control signal to infuse the medical liquid D according to a preset period.

For example, the processor 310 may be previously configured to infuse the medical liquid D in N-hour units, and in this case, the processor 310 may generate the control signal to control the first electrode 320 to apply the voltage to the variable member 250 every N hours.

**In** a selective embodiment, the processor 310 may generate the control signal to infuse the medical liquid D at a preset time.

**In** a selective embodiment, the processor 310 may monitor a state of contact between the first electrode 320 and the infusion needle portion 210 in real time.

For example, when a defect occurs in which the first electrode 320 does not contact the infusion needle portion 210, the control signal for infusion of the medical liquid D may be generated and thus the amount of the medical liquid D accommodated in the needle groove portion 111 may not be reduced, whereby the processor 310 may detect a poor contact state by monitoring the amount of the medical liquid D accommodated in the needle groove portion 111.

The processor 310 may monitor the amount of the medica liquid D accommodated in the needle groove portion 111 and the body data of the user to control the amount of the voltage applied to the variable member 250 from the first electrode 320, a time to apply the voltage to the variable member 250, etc.

As the substrate 300 applies or removes the voltage to the needle assembly 200, the medical liquid infusion apparatus 1 according to an embodiment of the present disclosure may control infusion of the medical liquid D to the user.

In addition, as the substrate 300 may receive user's body data from the needle assembly 200 to control the voltage applied to the needle assembly 200, a medical liquid D infusion environment optimized for a user's body state may be provided.

Hereinbelow, a configuration, operating principles, and effects of a medical liquid infusion apparatus 2 according to another embodiment of the present disclosure will be described. FIG. 8 is a bottom perspective view showing a medical liquid infusion apparatus according to another embodiment of the present disclosure.

Referring to FIG. 8, the medical liquid infusion apparatus 2 according to another embodiment of the present disclosure may include a body portion, a needle assembly 200', a substrate, a pressure generation unit, and a power supply unit.

Referring to FIG. 8, the needle assembly 200' may include an infusion needle portion 210' and a sensing needle portion 230'. The infusion needle portion 210' and the sensing needle portion 230' may be connected to the body portion, and may be provided in plural.

The plurality of infusion needle portions 210' may have a preset spacing in a radial direction from the center of the body portion, more specifically, a body housing 110', and may be arranged in a line.

The plurality of sensing needle portions 230' may be arranged in a line in a preset direction, and more specifically, may be arranged in a line in a circumferential direction with respect to the center of the body housing 110' on a surface of the body housing 110'.

As a result, the sensing needle portion 230' may obtain user's body data from an outer side of the infusion needle portion 210' that infuses medical liquid to the user.

Except that the body portion, more specifically, the body housing 110' is formed in a cylindrical shape, the plurality of infusion needle portions 210' may be arranged in a line in a radial direction from the center of the body housing 110', and the plurality of sensing needle portions 230' are arranged in a line in a radial direction, having a preset distance from the center of the body housing 110', the medical liquid infusion apparatus 2 according to another embodiment of the present disclosure have the same configuration, operating principles, and effects as those of the variable member 250, the substrate 300, the pressure generation unit 400, and the power supply unit 500 of the medical liquid infusion apparatus 1 according to an embodiment of the present disclosure, and thus will be described in detail in a redundant range.

The spirit of the present disclosure should not be determined by being limited to the above-described embodiments, and not only the claims to be described later, but also any range equivalent to or equivalently changed from the claims falls within the scope of the spirit of the present disclosure.

### Industrial Applicability

According to the present disclosure, a medical liquid infusion apparatus may be provided. Moreover, embodiments of the present disclosure may be applied to industrial devices that infuse medical liquid into a patient's body.

## Claims

1. A medical liquid infusion apparatus comprising:
a body portion in which a plurality of needle hole portions are formed;
a needle assembly connected to the needle hole portions and being capable of contacting a user's body;
a substrate capable of applying voltage to the needle assembly; and
a pressure generation unit configured to provide pressure to discharge medical liquid accommodated in the needle assembly to outside.

2. The medical liquid infusion apparatus of claim 1, wherein the needle assembly comprises:
an infusion needle portion capable of infusing medicine into a user's body; and
a sensing needle portion separated from the infusion needle portion and capable of collecting user's body data.

3. The medical liquid infusion apparatus of claim 2, wherein the needle assembly further comprises a variable member capable of contacting the substrate and being shape-deformed by receiving voltage from the substrate.

4. The medical liquid infusion apparatus of claim 3, wherein the variable member receives voltage to communicate the infusion needle portion with the pressure generation unit.

5. The medical liquid infusion apparatus of claim 3, wherein the substrate comprises:
a processor configured to receive the body data from the sensing needle portion and generate a control signal; and
a first electrode configured to receive the control signal and apply voltage to the variable member.

6. The medical liquid infusion apparatus of claim 5, wherein the processor is further configured to receive an electric signal from an external user terminal and control the first electrode to selectively apply voltage to the variable member based on the electric signal.
